# EUROPÄISCHE PATENTSCHRIFT

(11) **EP 0 861 636 B1**
(45) Veröffentlichungstag und Bekanntmachung des Hinweises auf die Patenterteilung: **22.10.2003**
(21) Anmeldenummer: 98103359.0
(22) Anmeldetag: 26.02.1998
(51) Int. Cl.: A61B 17/70

(54) **Vorrichtung zum Positionieren von Knochen und/oder Knochenfragmenten**
Device for positioning bone and/or bone fragments
Dispositif pour la mise en place des os et/ou des fragments d'os

(30) Priorität: 26.02.1997 DE 19707677
(43) Veröffentlichungstag der Anmeldung: 02.09.1998
(60) Teilanmeldung: 03018817.1
(73) Patentinhaber: Deutsches Zentrum für Luft- und Raumfahrt e.V., 53175 Bonn (DE)
(72) Erfinder: Balazs, Matthias,, 82284 Grafrath (DE); Wolf, Oleg, Dr., 13125 Berlin-Karow (DE)
(74) Vertreter: von Kirschbaum, Albrecht, Dipl.-Ing.

(56) Entgegenhaltungen:
- WO-A-91/06254
- WO-A-96/29947
- DE-A- 2 747 312
- DE-A- 19 512 709
- US-A- 4 946 458

## Beschreibung

Die Erfindung betrifft eine Vorrichtung zum Positionieren und Fixieren von Knochen und/oder Knochenfragmenten nach dem Oberbegriff des Anspruchs 1.

Eine derartige Vorrichtung ist aus WO 96/29 947 bekannt. Diese bekannte Vorrichtung weist eine Pedikelschraube, die aus einem Schraubekörper mit Außengewinde und einer Hülse mit zum Außengewinde passenden Innengewinde besteht, und ein Verbindungselement in Form einer sogenannten Kugelkopfschraube auf. Dies bedeutet zum einen, vor oder während einer Operation müssen diese Teile der Pedikelschraube montiert d.h. miteinander verschraubt werden. Ferner ist durch das Vorsehen von zwei Gewinden keine eindeutige und damit zielgerichtete Montage der Pedikelschraube, der Kugelkopfschraube und der auf die Pedikelschraube geschraubten Hülse bezüglich einer Lochplatte mit Hilfe einer Sicherungsmutter möglich.

Wird die Sicherungsmutter fest angezogen, ist nicht eindeutig, welches der beiden Gewinde dadurch festgelegt wird. So kann beispielsweise sowohl eine Verschraubung zwischen der Pedikelschraube und der Hülse als auch eine Verschraubung zwischen einem Kugelstift und der Sicherungsmutter festgelegt werden, ohne daß die jeweils andere Verschraubung ordnungsgemäß festgezogen sein muß. Somit weiß ein operierender Chirurg bei der aus WO 96/29 947 bekannten Vorrichtung nicht genau, ob das Implantat wirklich festsitzt oder ob es unter Umständen noch locker ist.

Ferner kann bei dieser bekannten Vorrichtung eine Pedikelschraube nicht mittels eines Werkzeug in einen Knochen eingeschraubt werden. Darüber hinaus kann bei der bekannten Vorrichtung beim Einschrauben einer Sicherungsmutter das Anzugsmoment nicht eindeutig abgestützt werden; das bedeutet, sowohl die Pedikelschraube als auch gegebenenfalls der Kugelkopf des Verbindungselements können verdreht werden. Dadurch kann jedoch das Implantat eine präzise vorherbestimmte Ausrichtung verlieren.

Weiterhin ist bei der bekannten Vorrichtung nachteilig, dass sich die Verschraubung in unerwünschter Weise lösen bzw. gegebenenfalls sogar festziehen kann; hierdurch wird dann entweder ein definiertes Festziehen der Schraubverbindung unmöglich oder das Verbindungselement mit Kugelkopf wird in einer unerwünschten Richtung verklemmt, so dass dadurch eine gezielte Ausrichtung verhindert ist. Obendrein besitzt die Sicherungsmutter keine zu einer Öffnung in der Lochplatte korrespondierende Profilierung, so dass eine eindeutige Ausrichtung bzw. Orientierung zwischen Sicherungsmutter und der Lochplatte nicht einwandfrei gewährleistet ist.

Aus 195 12 709 A1 ist eine weitere Vorrichtung bekannt, bei welcher Elemente der Vorrichtung wiederum in der richtigen Reihenfolge zusammensetzt werden müssen. Obendrein ist die Handhabung dieser Vorrichtung sehr umständlich, da ein frei präparierten Knochenbereich nur eingeschränkt zugänglich ist.

Aus DE 27 47 312 A ist zum Verbinden von Knochenteilen eine Schraube mit abgerundeten Flanken bekannt.

Darüber hinaus ist die mechanische Festigkeit und damit die Sicherheit des Implantats durch starke Sprünge im Querschnitt eingeschränkt. Obendrein sind die Wandstärken der bekannten Vorrichtung schwach ausgelegt und starke Kraftumlenkungen erforderlich. Darüber hinaus erfordert bei der bekannten Vorrichtung die Werkstoffbelastung aufgrund von mechanischen Spannungen beachtliche Querschnitte, wodurch die Vorrichtung insgesamt erheblich voluminöser ausgeführt werden muß.

Aufgabe der Erfindung ist es daher, Vorrichtungen zum Positionieren und Fixieren von Knochen und/oder Knochenfragmenten zu schaffen, mit denen in allen vorrichtungsbedingt möglichen Lagen Knochen und/oder Knochenfragmente bei gleichbleibender Festigkeit ohne Auftreten unerwünschter Vorspannungen fixierbar und positionierbar sind, wobei ein Vormontieren einzelner Vorrichtungsteile entfällt.

Gemäß der Erfindung ist diese Aufgabe bei einer Vorrichtung zum Positionieren und Fixieren von Knochen und/oder Knochenfragmenten durch die Merkmale im kennzeichnenden Teil des Anspruchs 1 gelöst. Vorteilhafte Weiterbildungen sind Gegenstand von auf Anspruch 1 unmittelbar oder mittelbar rückbezogenen Ansprüchen.

Bei einer Ausführungsform der erfindungsgemäßen Vorrichtung hat der sich an einen zylindrischen Kern mit Schraubgewinde konstanten Durchmessers anschließende Abschnitt eines Befestigungselements ein außen als Sechskant ausgebildetes Kopfteil mit einer in dessen Inneren ausgebildeten kugelsegmentförmigen Aussparung zur Aufnahme eines komplementären Endteils des Verbindungselements. Die kugelsegmentförmige Aussparung ist an der dem Schraubgewinde abgewandten Seite offen. An der offenen Seite der kugelsegmentförmigen Aussparung ist eine sich zur Aussparung hin verjüngende kegelstumpfförmige Aussparung ausgebildet, deren fiktive Kegelspitze einen Winkel von vorzugsweise 30° bis 40° aufweist, wodurch der Schwenkbereich des Verbindungselements bezüglich des Befestigungselements vorgegeben ist.

Gemäß der Erfindung entfällt entweder ein Vormontieren oder insbesondere ein Zusammensetzen der eine Haltevorrichtung bildenden Teile durch einen operierenden Arzt während der Operation. Nachdem die einzelnen Befestigungsschrauben der benötigten Haltevorrichtungen in vorbereitete Pedikelkanäle, in welche zweckmäßigerweise ein dem Schraubgewinde der Befestigungsschrauben entsprechendes Gewinde geschnitten ist, eingeschraubt sind, brauchen die Halterungsteile lediglich noch durch die Löcher, vorzugsweise in Form von Langlöchern der als Protheseplatte dienenden Lochplatte gesteckt, die Sicherungsschraube(n) aufgeschraubt und mit einem Drehmomentschlüssel angezogen zu werden.

Ferner ist die Lochplatte, die mindestens zwei Langlöcher aufweist, bezüglich der Haltevorrichtungen verschiebbar, so dass beim Fixieren der Schrauben an der Lochplatte keine Vorspannungen auftreten, die über die Befestigungsschraube in die Knochen und/oder Knochenfragmente eingeleitet würden. Da das Verschwenken der Schrauben unabhängig vom Verschieben der Haltevorrichtungen in den Langlöchern der Lochplatte erfolgt, ist die Handhabbarkeit der Vorrichtung erleichtert.

Um den Kopfteil der Befestigungsschraube zuverlässig fixieren zu können, ist die Gewindestange an dem dem Kopfteil der Befestigungsschraube zugewandten Ende entsprechend der Wölbung des kugelförmigen Kopfteils konkav ausgebildet.

Ferner sind die oberen bzw. unteren Kanten der Langlöcher in den Lochplatten angefast bzw. auch kugelsegmentförmig ausgebildet. Der Kopfbereich der Schraube und der der Lochplatte zugewandte Bereich der auf das Verbindungselement geschraubten Sicherungsmutter(n) weisen jeweils eine zu den Kanten der Langlöcher passende Form auf.

Somit ist sichergestellt, dass beim Fixieren der Schraube durch Anziehen der Sicherungsmutter(n) die Mittelachse des Verbindungselements stets senkrecht zu der Lochplatte ausgerichtet ist. Daher besteht zwischen Mutter und Lochplatte bzw. zwischen Schraubenkopf und Lochplatte bei der erstgenannten Ausführung immer Linienberührung, so dass an der Lochplatte eine gleichmäßige Lastverteilung auftritt.

Vorzugsweise sind gemäß der Erfindung sämtliche oder einzelne der Berührflächen zwischen den einzelnen Elementen der Vorrichtung statistisch profiliert oder mit feinen Querrillen versehen. Somit besteht zwischen den einzelnen Elementen ein sicherer Halt, da die Reibung durch Formschluss erhöht ist.

Nachfolgend wird die Erfindung anhand einer bevorzugten Ausführungsform unter Bezugnahme auf die anliegenden Zeichnungen im einzelnen erläutert. Es zeigen:
- Fig.1a: eine Schnittansicht der bevorzugten Ausführungsform entlang der Linie I-I in Fig.1b;
- Fig.1b: eine Schnittansicht der bevorzugten Ausführungsform entlang der Linie II-II in Fig.1a;
- Fig.1c: eine Vorderansicht der bevorzugten Ausführungsform; und
- Fig. 2a: bis 2d bevorzugte Ausführungsformen von Lochplatten, die zum Positionieren und Fixieren von Knochen und/oder Knochenfragmenten verwendbar sind.

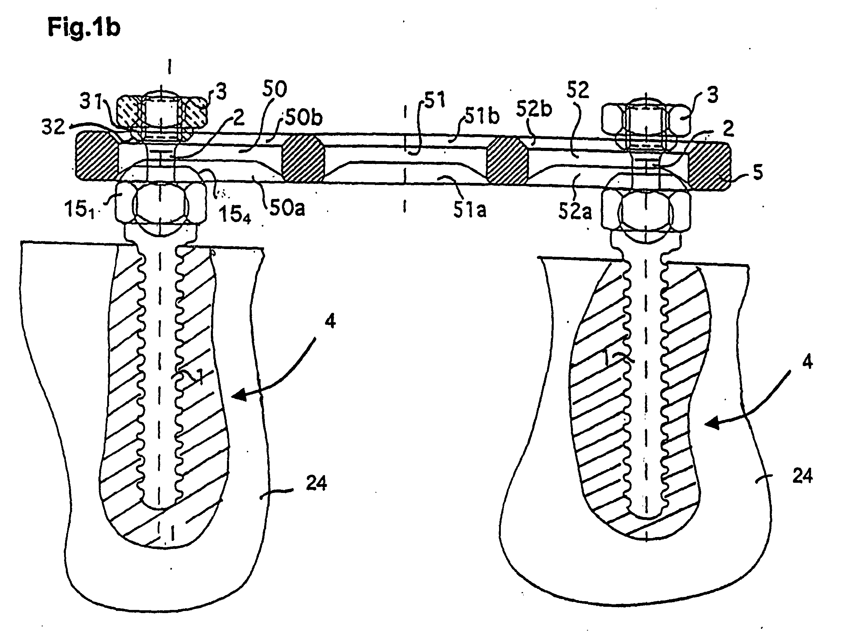

In Fig.1b ist eine als Prothesenplatte dienende, im Schnitt dargestellte Lochplatte 5 dargestellt, die in Fig.1b beispielsweise drei Langlöcher 50 bis 52 aufweist. In die beiden äußeren Langlöcher 50 und 52 der Lochplatte 5 ist je eine Haltevorrichtung 4 eingesetzt, deren wesentliche Elemente, in Fig.1b von oben nach unten, eine Sicherungsmutter 3, ein Verbindungsteil 2 und eine Befestigungsschraube 1 sind. Die Befestigungsschrauben 1 der beiden Haltevorrichtungen 4 sind in von einem Operateur vorher in schematisch angedeutete Wirbel 24 ausgebildete, in Fig.1b nicht näher bezeichnete Kanäle eingeschraubt.

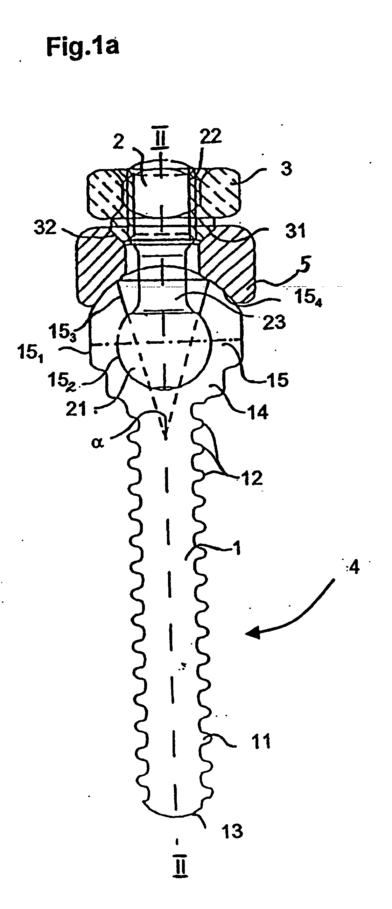

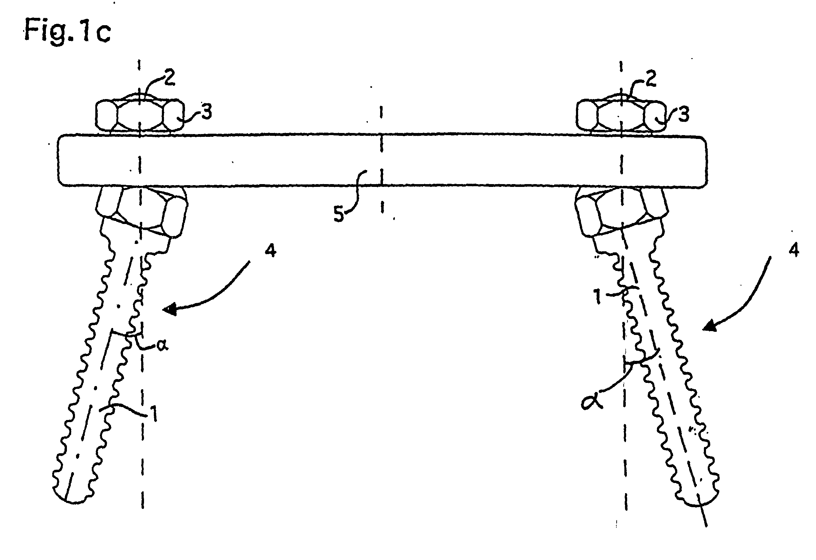

Unter Bezugnahme auf Fig.1a wird nunmehr eine bevorzugte Ausführungsform der Haltevorrichtung 4 anhand einer Schnittansicht entlang der Linie I-I in Fig.1b beschrieben. In Fig.1a weist die Befestigungsschraube 1 der Haltevorrichtung 4 einen zylindrischen Kern mit einem Schraubgewinde 11 auf, welches somit einen konstanten Außendurchmesser hat. Die Flanken des Schraubgewindes 11 weisen abgerundete Flankenenden 12 auf. Das in Fig.1 untere freie Ende 13 der Schraube 1 ist ebenfalls abgerundet.

Am in Fig.1a oberen Ende des Schraubgewindes 11 geht dieses in einen ringförmigen Ansatz 14 über, der vorzugsweise zylindrisch ausgebildet ist und dessen Außendurchmesser größer als der Außendurchmesser des Schraubgewindes 11 ist.

Der ringförmige Ansatz 14 geht in einen Kopfteil 15 über, dessen Außenabmessung größer als derjenige des ringförmigen Ansatzes ist und vorzugsweise außen als Sechskant 15₁ ausgebildet ist, um ein geeignetes Werkzeug, beispielsweise einen handelsüblichen Maulschlüssel ansetzen zu können. Im Inneren weist der Kopfteil 15 eine kugelförmige Aussparung 15₂ auf. Die kugelförmige Aussparung 15₂ geht in eine zur gestrichelt wiedergegebenen Mittenachse der Schraube 1 symmetrische kegelstumpfförmige Aussparung 15₃ über, die in Fig.1 nach oben offen ist.

Der kleinere Durchmesser der kegelstumpfförmigen Aussparung 15₃ grenzt an den oberen Teil der kugelförmigen Aussparung 15₂ an. Die in der Schnittansicht der Fig.1a gestrichelt wiedergegebenen Verlängerungen der kegelstumpfförmigen Aussparung 15₃ schließen vorzugsweise einen spitzen Winkel α von 30° - 40° ein. Der Sechskant 15₁ des Kopfteils 15 geht in einen kugelschalenförmigen Abschnitt 15₄ über.

Der Durchmesser der kugelförmigen Aussparung 15₂ im Kopfteil 15 ist größer als der angrenzende kleinere Durchmesser der kegelstumpfförmigen Aussparung 15₃. Somit entsteht, wie Fig.1a deutlich zu entnehmen ist, an dem Übergangsbereich zwischen der kegelstumpfförmigen Aussparung 15₃ und der kugelförmigen Aussparung 15₂ eine Unterschneidung.

In die kugelförmige Aussparung 15₂ des Kopfteils 15 der Befestigungsschraube 1 ist in Fig.1a ein zu der kugelförmigen Aussparung 15₂ komplementärer kugelförmiger Endteil 21 des Verbindungsteils 2 eingesetzt. Das dem kugelförmigen Endteil 21 des Verbindungsteils 2 abgewandte Ende ist kreiszylindrisch ausgebildet und ist mit einem vorzugsweise als Feingewindeprofilierung ausgebildeten Außengewinde 22 versehen.

Zwischen dem kugelförmigen Endteil 21 und dem mit dem Außengewinde 22 versehenen zylindrischen Bereich des Verbindungsteils 2 ist ein gegenüber dem zylindrischen Endteil verjüngter Schaftteil 23 ausgebildet. Aufgrund der Verjüngung handelt es sich bei dem Schaftteil 23 um einen in engen Grenzen dehnbaren Schaftteil.

Um den kugelförmigen Endteil 21 des Verbindungsteils 2 in die kugelförmige Aussparung 15₂ des Kopfteils 15 der Befestigungsschraube 1 einbringen zu können, muß der Kopftel 15 aus fertigungstechnischen Gründen aus zwei Einzelteilen hergestellt werden. Hierbeikann die Trennlinie zwischen den beiden den Kopfteil 15 bildenden Einzelteilen, beispielsweise die strichpunktiert eingetragenen Trennlinie sein. Nach dem Einsetzen des kugelförmigen Endteils 21 des Verbindunsgteils 2 in den unteren, mit der Befestigungsschraube 1 eine Einheit bildenden Teil des Kopfteils wird dieser mit dem oberhalb der strichpunktierten Linie dargestellten Teil des Kopfteils 15 fest verbunden, beispielsweise verschweißt oder gegebenenfalls auch verklebt, so daß der Kopfteil 15 anschließend eine in Fig.1a dargestellte Einheit mit der Befestigungsschraube bildet.

Da der kugelförmige Endteil 21 des Verbindungsteils 2 und die kugelförmige Aussparung 15₂ im Kopfteil 15 komplementär ausgebildet sind, ist der Verbindungsteil 2 in dem Kopfteil 15 zum einen um die gestrichelt wiedergegebene Achse drehbar und zum anderen in dem durch die kegelstumpfförmige Aussparung 15₃ festgelegten Bereich bezüglich der gestrichelt wiedergegebenen.Mittenachse auch schwenkbar. (Siehe hierzu beispielsweise Fig.1c) Der Winkelbereich, in welchen der Verbindungsteil 2 gegenüber der Befestigungsschraube 1 schwenkbar ist, entspricht dem in Fig.1a mit α bezeichneten Winkelbereich und liegt bei der Ausführungsform der Fig.1a in der Größenordnung von 30° bis 35°.

Die Sicherungsmutter 3, die vorzugsweise als Sechskantmutter ausgebildet ist, weist ein der Feingewindeprofilierung des Außengewindes 22 des Verbindungsteils 2 entsprechendes Gewinde und auf der in Fig.1a unteren Seite einen ringförmigen Ansatz 31 mit einer Anfasung 32 auf.

In Fig.1b, welche eine Schnittansicht entlang der Linie II-II in Fig.1a ist, ist die als Prothesenplatte dienende Lochplatte 5 teilweise im Schnitt dargestellt. Wie bereits erwähnt, weist die Lochplatte 5 in Fig.1b drei Langlöcher 50 bis 52 auf. Die in Fig.1b oberen Kanten 50b bis 52b sind passend zu der Anfasung 32 des ringförmigen Ansatzes 31 der Sicherungsmutter 3 abgeschrägt.

Die Kanten 50a bis 52a der Langlöcher 50 bis 52 sind so ausgerundet, daß deren Ausrundung dem kugelförmigen Endabschnitt 15₄ des Kopfteils 15 am oberen Ende der Befestigungsschraube 1 entspricht. Der Radius der konkav ausgebildeten Kantenbereiche 50a bis 52a der drei Langlöcher 50 bis 52 entspricht somit dem Radius des kugelförmigen Abschnitts 15₄ des Kopfteils 15. Aufgrund der konkaven Ausbildung der Kantenbereiche 50a bis 52a ist ein entsprechendes Kippen bzw. Neigen der Befestigungsschrauben 1 bezüglich der als Prothesenplatte dienenden Lochplatte 5 ermöglicht (siehe Fig.1c).

Die als Prothesenplatte dienende Lochplatte wird an einem Pedikel wie folgt befestigt: Zunächst wird von dem Operateur ein Pedikelkanal in einen Wirbel 24 eingebracht. Zweckmäßigerweise sollte in dem Pedikelkanal auch ein dem Schraubgewinde 11 der einzubringenden Befestigungsschraube 1 entsprechendes Gewinde geschnitten werden. Durch Ansetzen eines Werkzeugs, beispielsweise eines Maulschlüssels, an dem Sechskant 15₁ am Kopfteil 15 der Befestigungsschraube 1 wird diese zusammen mit dem in den Kopfteil 15 eingesetzten Verbindungsteil 2 so weit eingedreht, dass sich der Kopfteil 15 und der aus diesem vorstehende Verbindungsteil 2 in der richtigen Höhenlage befinden.

Anschließend wird der Verbindungsteil 2 der Haltevorrichtung 4 durch eines der Langlöcher 50 bis 52 der Lochplatte 5 gesteckt. Hierauf wird die Sicherungsmutter 3 auf das Außengewinde 22 des Verbindungsteils 2 aufgeschraubt und mittels eines Werkzeugs, vorzugsweise eines Drehmomentenschlüssels so weit angezogen, bis das erforderliche und zulässige Drehmoment erreicht ist.

Die gleiche Prozedur wird mit der oder den übrigen Halterungsvorrichtungen durchgeführt, so dass dann über die als Prothesenplatte dienende Lochplatte 5 eine genaue Positionierung und Fixierung von Knochen, beispielsweise Wirbeln und/oder auch von Knochenfragmenten erreicht ist.

Der Unterschied zwischen der Schnittansicht in Fig.1b und der Vorderansicht in Fig.1c besteht im wesentlichen darin, dass in Fig.1b die gestrichelt wiedergegebenen Mittenachsen der Befestigungsschrauben 1 und der Verbindungsteile 2 der beiden in Fig.1a und 1b dargestellten Haltevorrichtungen 4 fluchten, während in Fig.1c die dort strichpunktiert eingetragene Mittenachse der Befestigungsschrauben 1 mit der gestrichelt wiedergegebenen Mittenachse der Verbindungsteile 2 einen spitzen Winkel α in der Größenordnung von etwa 15 bis 20° miteinander einschließen. Hierbei soll es sich bei dem Neigungswinkel α in Fig.1c um den maximal möglichen Schwenkbereich handeln, welcher mit der in Fig.1a wiedergegebenen Haltevorrichtung 4 möglich ist.

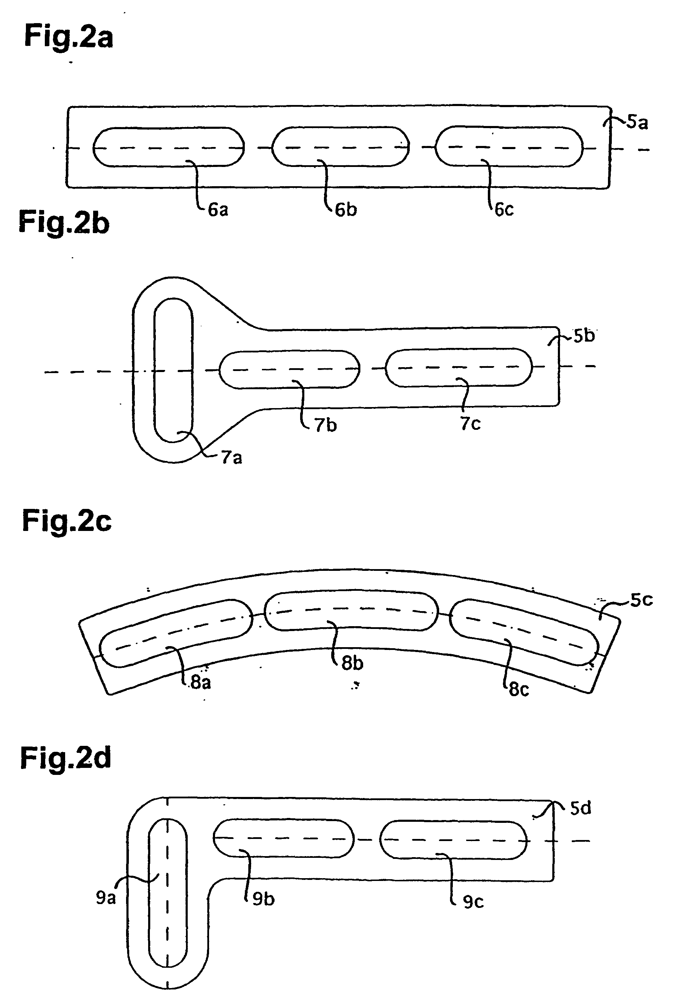

In Fig.2a bis 2d sind beispielhaft mögliche Ausführungsformen von Lochplatten 5a, 5b, 5c und 5d dargestellt. Fig.2a zeigt eine Lochplatte 5a, die drei Langlöcher 6a, 6b und 6c aufweist, die auf einer gestrichelt wiedergegebenen Mittellinie angeordnet sind und, wie auch in Fig.3a dargestellt, unterschiedliche Längen haben können.

In Fig.2b ist eine T-förmige Lochplatte 5b dargestellt, die zwei in Reihe angeordnete, in Fig.2b gleich große Langlöcher 7b und 7c und ein zu diesen beispielsweise senkrecht angeordnetes Langloch 7a aufweist.

In Fig. 2c ist eine bogenförmige Lochplatte 5c dargestellt, die beispielsweise drei gleichgroße Langlöcher 8a, 8b und 8c aufweist, die entlang einer gestrichelten gekrümmten Linie ausgerichtet sind.

In Fig. 2d ist eine L-förmige Lochplatte 5d dargestellt, die zwei in Reihe hintereinander angeordnete, in Fig.3d gleich große Langlöcher 9b, 9c sowie ein zu diesen senkrechtes Langloch 9a aufweist.

Ferner sind auch andere beliebige Kombinationen von unterschiedlich bemessenen Langlöchern denkbar. Die in Fig.2a bis 2d dargestellten Lochplatten sind als in sich eben dargestellt. Die einzelnen Lochplatten können auch so vorgeformt sein, dass beispielsweise ihre beiden Endbereiche nach oben abgewinkelt sind oder ein Endbereich nach unten und der andere nach oben abgewinkelt ist oder überhaupt nur ein Endbereich abgewinkelt bzw. gekröpft ist, während der andere Endbereich eben ausgeführt ist.

## Patentansprüche

1. Vorrichtung zum Positionieren und Fixieren von Knochen und/oder Knochenfragmenten, mit einer als Prothesenplatte dienenden Lochplatte (5) und Haltevorrichtungen (4), bestehend jeweils aus einem Befestigungselement (1) mit Schraubgewinde (11), einem Sicherungsmittel (3)und einem Verbindungselement (2) mit Außengewinde (22) an einem Ende und einem kugelförmigen Endteil (21) am anderen Ende, so dass die Haltevorrichtungen (4) mittels der Sicherungsmittel (3) an der Lochplatte (5) befestigbar und fixierbar sind, **dadurch gekennzeichnet, dass**
jedes Befestigungselement einen zylindrischen Kern mit Schraubgewinde (11) konstanten Durchmessers und daran anschließend ein außen als Sechskant (15₁) ausgebildetes, aus zwei Einzelteilen zusammengesetztes Kopfteil (15) hat,
in dessen Inneren eine zum Unterbringen des kugelförmigen Verbindungselement-Endteils (21) komplementäre, kugelförmige Aussparung (15₂) dadurch geschaffen ist, dass nach Einbringen des kugelförmigen Verbindungselement-Endteils (21) in das an das Schraubgewinde (11) anschließende Einzelteil des Kopfteils dieses mit dem anderen Einzelteil des Kopfteils (15) durch Verschweißen oder Verkleben fest verbunden wird, so dass das Verbindungselement (2) und das Befestigungselement (1) eine Einheit bilden,
die kugelförmige Aussparung (15₂) an der dem Schraubgewinde (11) abgewandten Seite offen ist, und
an der offenen Seite der kugelförmigen Aussparung (15₂) eine sich zu dieser Aussparung hin verjüngende, kegelstumpfförmige Aussparung (15₃) ausgebildet ist, deren fiktive Kegelspitze einen Winkel von 30° bis 40° aufweist, wodurch der Schwenkbereich des Verbindungselements (2) bezüglich des Befestigungselements (1) festgelegt ist.

2. Vorrichtungen nach Anspruch 1, , **dadurch gekennzeichnet, dass** zwischen dem kugelförmigen Endteil (21) und dem Gewindebereich (22) des Verbindungsteils (2) ein dehnbarer Schaftteil (23) vorgesehen ist.

3. Vorrichtung nach Anspruch 1, **dadurch gekennzeichnet, dass** die äußeren Flankenenden (12) des Schraubgewindes (11) des Befestigungselements (1) abgerundet sind.

4. Vorrichtung nach Anspruch 1, **dadurch gekennzeichnet, dass** das Befestigungselement (1) an einem Ende (13) abgerundet ist.

5. Vorrichtung nach Anspruch 1, **dadurch gekennzeichnet, dass** der Kopf (15) des Befestigungselements (1) eine statistisch rauhe Profilierung oder feine Querrillen aufweist.

6. Vorrichtung nach Anspruch 1, **dadurch gekennzeichnet, dass** Kantenbereiche von Langlöchern (50 bis 52) der Lochplatte (5) entsprechend der Krümmung des Kopfes (15) des Befestigungselements (1) ausgebildet sind.

7. Vorrichtung nach den Ansprüchen 1 und 6, **dadurch gekennzeichnet, dass** die einer Sicherungsmutter (3) zugewandten Kanten der Langlöcher (50 bis 52) eine Anfasung aufweisen und die Sicherungsmutter (3) einen ringförmigen Ansatz (31) mit Anfasung (32) aufweist.

8. Vorrichtung nach einem der vorhergehenden Ansprüche, **dadurch gekennzeichnet, dass** alle Elemente der Vorrichtung aus demselben Werkstoff hergestellt sind wie Implantatstähle, CoCrMo, Ti6-A14V oder andere zugelassene Implantatwerkstoffe auf metallischer Basis oder phaserverstärkte Implantatkunststoffe, beispielsweise kohlefaserverstärktes Duromer verwendet sind.

## Claims

1. A device for positioning and fixating bones and/or bone fragments comprising a slotted plate (5) serving as a prosthetic plate and anchoring devices (4) each consisting of a fastener element (1) including a screw thread (11), a locking means (3) and a connecting element (2) with a male thread (22) at one end and a spherical end part (21) at the other end, so that said anchoring devices (4) can be secured and fixated to said slotted plate (5) by means of said locking means (3), **characterized in that**
each fastener element (1) comprises a cylindrical core with a screw thread (11) of constant diameter and adjoining thereto a head part (15) configured externally hexagonal (15₁), composed of two component parts, in the interior of which a complementary spherical recess (15₂) for seating said spherical end part (21) is provided by, following application of said spherical end part (21) to said component adjoining said screw thread (11) of said head part (15), it being fixedly connected to the other component of said head part (15) by welding or adhesive bonding so that said connecting element (2) and said fastener element (1) form a single unit
said spherical recess (15₂) is open at the end facing away from said screw thread (11), and
at the open end of said spherical recess (15₂) a recess (15₃) in the shape of a truncated cone tapered towards said recess is configured, the imaginary conical apex of which has an angle in the range 30° to 40° defining the pivoting range of said connecting element (2) relative to said fastener element (1).

2. The device as set forth in claim 1, **characterized in that** provided between said spherical end part (21) and threaded portion (22) of said connecting part (2) is an expandable shank part (23).

3. The device as set forth in claim 1, **characterized in** the outer flank ends (12) of said screw thread (11) of said fastener element (1) are rounded.

4. The device as set forth in claim 1, **characterized in that** said fastener element (1) is rounded at one end (13).

5. The device as set forth in claim 1, **characterized in that** said head (15) of said fastener element (1) comprises a statistically rough profile or fine transverse grooves.

6. The device as set forth in claim 1, **characterized in that** edge portions of said slots (50 to 52) in said slotted plate (5) are configured to conform with the curvature of said head (15) of said fastener element (1).

7. The device as set forth in claim 1 and 6, **characterized in that** the edges of said slots (50 to 52) facing a locknut (3) are chamfered and said locknut (3) comprises an annular lug (31) including a land (32).

8. The device as set forth in any of the preceding claims, **characterized in that** all elements of said device are made of the same material such as implant steels, CoCrMo, Ti6-A14V or other metal-based approved implant materials or fiber-reinforced implant plastics, for example carbon fiber reinforced thermosetting plastic.

## Revendications

1. Dispositif pour positionner et pour fixer des os et/ou des fragments osseux, comprenant une plaque à trous (5) servant de plaque de prothèse et des dispositifs de maintien (4), chacun constitué d'un élément de fixation (1) avec taraudage (11), d'un organe de blocage (3) et d'un élément de liaison (2) avec filetage (22) à une extrémité et avec une partie terminale de forme sphérique (21) à l'autre extrémité, de sorte que les dispositifs de maintien (4) sont susceptibles d'être immobilisés et fixés sur la plaque à trous (5) au moyen de l'organe de blocage (3), **caractérisé en ce que** :
chaque élément de fixation possède un noyau cylindrique avec un taraudage (11) de diamètre constant et une partie de tête (15) qui s'y raccorde et réalisée à l'extérieur sous forme de relief hexagonal (15₁) composé de deux pièces individuelles, partie de tête à l'intérieur de laquelle est ménagé un évidement (15₂) de forme sphérique complémentaire destiné à loger la partie terminale (21) sphérique de l'élément de liaison, par le fait qu'après l'introduction de la partie terminale sphérique (21) de l'élément de liaison dans la pièce individuelle de la partie de tête qui fait suite au taraudage (11) celle-ci est fermement reliée à l'autre pièce individuelle de la partie de tête (15) par soudage ou par collage, de sorte que l'élément de liaison (2) et l'élément de fixation (1) forment une unité,
l'évidement de forme sphérique (15₂) est ouvert du côté détourné du taraudage (11), et
sur le côté ouvert de l'évidement (15₂) de forme sphérique est réalisé un évidement (15₃) de forme tronconique qui va en se rétrécissant vers cet évidement, dont la pointe conique fictive présente un angle de 30 à 40°, grâce à quoi la plage de pivotement de l'élément de liaison (2) par rapport à l'élément de fixation (1) est déterminée.

2. Dispositif selon la revendication 1, **caractérisé en ce qu'**il est prévu une partie de fut (23) extensible entre la partie terminale sphérique (21) et la zone filetée (22) de la pièce de liaison (2).

3. Dispositif selon la revendication 1, **caractérisé en ce que** les extrémités extérieures (12) des flancs du taraudage (11) de l'élément de fixation (1) sont arrondies.

4. Dispositif selon la revendication 1, **caractérisé en ce que** l'élément de fixation (1) est arrondi à une extrémité (13).

5. Dispositif selon la revendication 1, **caractérisé en ce que** la tête (15) de l'élément de fixation (1) présente un profilage statistiquement rugueux, ou des rainures transversales fines.

6. Dispositif selon la revendication 1, **caractérisé en ce que** des zones des arêtes de trous oblongs (50 à 52) de la plaque à trous (5) sont réalisées en correspondance de la courbure de la tête (15) de l'élément de fixation (1).

7. Dispositif selon les revendications 1 et 6, **caractérisé en ce que** les arêtes des trous oblongs (50 à 52) qui sont tournées vers un écrou de blocage (3) présentent un chanfrein, et **en ce que** l'écrou de blocage (3) comporte un talon annulaire (31) avec un chanfrein (32).

8. Dispositif selon l'une des revendications précédentes, **caractérisé en ce que** tous les éléments des dispositifs sont fabriqués avec le même matériau, comme des aciers pour implants, du CoCrMo, du Ti6-A14V, ou autres matériaux autorisés pour des implants, à base métallique ou de matières plastiques pour implants renforcées au moyen de fibres, en particulier des duromères renforcés par des fibres de carbone.
